# DEMANDE DE BREVET EUROPEEN

(11) **EP 4 484 578 A1**
(43) Date de publication de la demande: **01.01.2025**
(21) Numéro de dépôt: 23182506.8
(22) Date de dépôt: 29.06.2023
(51) Int. Cl.: C12Q 1/689, C12Q 1/04, C12Q 1/06, G01N 33/569

(54) **MÉTHODE POUR LA DÉTECTION ET LA CONFIRMATION DES ESCHERICHIA COLI PRODUCTRICE DE SHIGATOXINE ET/OU D'UNE E. COLI ENTÉROHÉMORRAGIQUE**

(71) Demandeur: BIOMÉRIEUX, 69280 Marcy L'Etoile (FR)
(72) Inventeur: CARRET, Justine, 38850 BILIEU (FR); DOMINGOS, Céline, 69270 FONTAINE SAINT MARTIN (FR); HAMON, Fabienne, 38140 APPRIEU (FR); JUNILLON, Thomas, 69290 CRAPONNE (FR); MALLEN, Benoit, 69290 GREZIEUX LA VARENNE (FR); ROGER-CARDOSO, Tamara, 38600 FONTAINE (FR)
(74) Mandataire: bioMérieux PI Groupement mandataires

(57) **Abrégé**

L'invention concerne une méthode de détection et de confirmation d'au moins une *Escherichia Coli* productrice de shigatoxine (STEC) susceptible d'être présente dans un échantillon comprenant des entérobactéries, comprenant les étapes suivantes :
- Effectuer une lyse de l'échantillon permettant la lyse des STEC afin d'obtenir une solution comprenant leurs acides nucléiques
- Mettre en contact la solution d'acides nucléiques avec des amorces permettant d'amplifier au moins le gène ou le fragment de gène stx1 et/ou stx2
- Si au moins un des gènes ou fragments des gènes stx1 et/ou stx2 est amplifié, une partie de l'échantillon est déposé sur un milieu réactionnel gélosé comprenant
■ au moins un inducteur de toxine,
■ au moins un conjugué agglutinant formé par au moins un partenaire de liaison spécifique de la protéine STX1 et/ou au moins un partenaire de liaison spécifique de la protéine STX2, couplé(s) à une nanoparticule.

- Détecter et confirmer la présence d'au moins une STEC par l'apparition d'un halo sur la gélose autour de ladite STEC.

## Description

### DOMAINE TECHNIQUE

La présente invention concerne le domaine du contrôle microbiologique au sens large, tel que le contrôle microbiologique d'un échantillon d'origine industrielle ou clinique. Plus particulièrement, la présente invention a trait à une méthode pour la détection, l'identification, le dénombrement et/ou l'isolement d'*Escherichia coli* productrice de shigatoxine et/ou d'une *E. coli* entérohémorragique.

### TECHNIQUE ANTERIEURE

Le contrôle microbiologique d'échantillons d'origines diverses requiert la mise en oeuvre de techniques qui permettent la détection - par exemple aux fins d'identification et/ou de dénombrement et/ou de caractérisations biochimiques - de micro-organismes et dont le rendu en termes de résultats doit être le plus rapide possible.

Dans le domaine médical, il est nécessaire de prévoir et diagnostiquer le risque infectieux : plus le diagnostic est rapide et précis, plus la prise en charge des malades est efficace et le risque de transmission minimisé. L'approche est similaire pour la santé animale dans le domaine vétérinaire.

Dans le domaine agro-alimentaire, la problématique est identique. Elle distingue cependant :
- les micro-organismes pathogènes tels que les *E. coli* productrices de shiga-toxines (STEC), *Salmonella, Listeria, Cronobacter, Bacillus, Staphylococcus* dont la recherche s'applique aux matières premières, produits intermédiaires, produits finis commercialisés, - les micro-organismes non pathogènes, utilisés comme indicateurs-qualité du processus de production, des matières premières aux produits finis, tout au long de la chaîne,
- les bactéries d'intérêt technologique telles que les ferments,
- les micro-organismes marqueurs de contamination.

La détection rapide et précise des contaminations présumées (au sein des lots alimentaires) permet de les contrôler et d'engager ainsi à bref délai des actions correctives.

Au cours de ces dernières années, de nombreux pays ont été touchés par des épidémies d' *E. coli* productrices de shigatoxines (STEC) ou d' *E. coli* Entérohémoragiques (EHEC). Les infections humaines causées par ces bactéries sont aujourd'hui reconnues comme un problème de santé publique majeur à l'échelle mondiale. La grande majorité des bactéries *E. coli* sont des souches commensales de l'Homme. Toutefois, certaines souches ont acquis des gènes et facteurs de virulence. Cela leur permet de coloniser l'appareil digestif, de contourner les défenses immunitaires et induire des dommages cellulaires, provoquant alors des symptômes chez l'être humain. Parmi ces souches STEC qui ont acquis le facteur de virulence stx, un pathovar particulier est mis en cause : celui des souches *E. coli* Entérohémoragiques. Les EHEC sont des souches représentant un sous-groupe des STEC, qui ont acquis le gène eae et provoquent un syndrome hémolytique et urémique. La possession de ces deux facteurs de virulence en simultané, stx et eae, rend ce pathovar très virulent pour l'Homme. Il existe d'autres facteurs de virulence tels que aggR, LT, ST, afaC, ipaH.

Généralement, l'analyse microbiologique se fait en deux temps. La première est une phase de détection qui peut faire appel à de nombreuses technologies telles que les milieux de culture, les immuno-essais, la biologie moléculaire. Au cours de cette étape de détection, il s'avère difficile de savoir si une même souche est porteuse d'un ou plusieurs facteurs de virulence.

Elle peut être suivie d'une phase de confirmation afin de confirmer la présence du pathogène recherché et répondre aux normes en vigueur dans ce domaine. L'étape de confirmation impose donc des étapes supplémentaires et nécessite une étape d'isolement de la bactérie recherchée.

Ainsi, les méthodes de l'art antérieur nécessitent une succession d'étapes d'amplification, d'immunoconcentration et de culture sur boite afin de détecter et de confirmer la présence d'un pathovar d'Escherichia coli virulent.

Il existe donc un réel besoin de mettre au point une méthode fiable et rapide de détection et de confirmation de pathovars d'*E*. *coli* virulent.

### RESUME DE L'INVENTION

La présente invention concerne une méthode de détection et de confirmation d'au moins une *Escherichia Coli* productrice de shigatoxine (STEC) susceptible d'être présente dans un échantillon comprenant des entérobactéries, comprenant les étapes suivantes :
- Effectuer une lyse de l'échantillon permettant la lyse des STEC afin d'obtenir une solution comprenant leurs acides nucléiques
- Mettre en contact la solution d'acides nucléiques avec des amorces permettant d'amplifier au moins le gène ou le fragment de gène stx1 et/ou stx2
- Si au moins un des gènes ou fragments des gènes stx1 et/ou stx2 est amplifié, une partie de l'échantillon est déposé sur un milieu réactionnel gélosé comprenant :
   ▪ au moins un inducteur de toxine,
   ▪ au moins un conjugué agglutinant formé par au moins un partenaire de liaison spécifique de la protéine STX1 et/ou au moins un partenaire de liaison spécifique de la protéine STX2, couplé(s) à une nanoparticule.
- Détecter et confirmer la présence d'au moins une STEC par l'apparition d'un halo sur la gélose autour de ladite STEC.

La méthode selon la présente invention présente l'avantage de confirmer la présence ou non d'une STEC directement sur le milieu réactionnel par la formation d'un halo autour de ladite souche.

Cette invention est particulièrement intéressante pour faciliter la détection et la confirmation de la présence des STEC ou des EHEC dans un échantillon polymicrobien. En effet, sans la présente invention qui permet de localiser la colonie d'intérêt, la méthode de référence ISO 16136 précise qu'il est nécessaire de tester jusqu'à 50 colonies par méthode moléculaire pour confirmer la présence d'un STEC. Au regard du nombre important de colonies sur la boite et la faible représentation du micro-organisme cible, la personne prélevant les colonies à des fins de confirmation, peut ne jamais prélever le micro-organisme cible. Ainsi la présente invention permet d'éviter les faux négatifs et permet un gain de temps dans l'exécution du contrôle microbiologique. Elle est particulièrement avantageuse pour des échantillons chargés en flore annexe, où la quantité importante de colonies sur les boîtes de Pétri peut entraîner un risque de faux négatifs.

Un autre mode de réalisation porte sur une approche de détection sérogroupale. Ainsi un autre mode de réalisation concerne une méthode de détection et de confirmation d'au moins une *Escherichia Coli* productrice de shigatoxine (STEC) comprenant les étapes suivantes :
- Effectuer une lyse de l'échantillon permettant la lyse des STEC afin d'obtenir une solution comprenant leurs acides nucléiques
- Mettre en contact la solution d'acides nucléiques avec des amorces permettant d'amplifier au moins le gène ou le fragment de gène stx1 et/ou stx2
- Si au moins un des gènes ou fragments des gènes stx1 et/ou stx2 est amplifié, la solution d'acides nucléiques est mise en contact avec des amorces permettant d'amplifier au moins le gène ou le fragment de gène des sérogroupes choisis parmi O26, O45, O80, O91, O103, O104, O111, O113, O121, O128ab, O145, O146, O157, O174
- Si au moins un des gènes ou fragments des gènes stx1 et/ou stx2 et au moins un sérogroupe choisi parmi O26, O45, O80, O91, O103, O104, O111, O113, O121, O128ab, O145, O146, O157, O174 sont amplifiés, une partie de l'échantillon est déposé sur un milieu réactionnel gélosé comprenant
   ∘ au moins un inducteur de toxine et
   ∘ au moins un conjugué agglutinant formé par au moins un partenaire de liaison spécifique de la protéine STX1 et/ou au moins un partenaire de liaison spécifique de la protéine STX2, couplé(s) à une nanoparticule.
- Détecter et confirmer la présence d'au moins une STEC par l'apparition d'un halo sur la gélose autour de ladite STEC.

L'avantage de cette méthode est que l'approche sérogroupale permet de renforcer la présomption qu'il s'agit d'une STEC d'un sérogroupe cible.

Préférentiellement, la méthode de détection et de confirmation d'au moins une *Escherichia Coli* productrice de shigatoxine (STEC) comprend l'étape suivante :
- si au moins un des gènes ou fragments des gènes stx1 et/ou stx2 et au moins un gène ou fragment de gène d'au moins un sérogroupe choisi parmi O26, O45, O80, O91, O103, O104, O111, O113, O121, O128ab, O145, O146, O157, O174. sont amplifiés, une partie de l'échantillon est déposé sur un milieu réactionnel comprenant
   ∘ au moins un inducteur de toxine et
   ∘ au moins un conjugué agglutinant formé par au moins un partenaire de liaison spécifique de STX1 couplé à une nanoparticule d'une première nature produisant un halo d'une première couleur lorsqu'il est agglutiné
   ∘ et/ou au moins un conjugué agglutinant formé par au moins un partenaire de liaison spécifique de STX2 couplé à une nanoparticule d'une première nature produisant un halo d'une première couleur lorsqu'il est agglutiné
   ∘ et au moins un conjugué agglutinant formé par au moins un partenaire de liaison spécifique dudit serogroupe identifié par amplification couplé à une nanoparticule d'une deuxième nature produisant un halo d'une deuxième couleur lorsqu'il est agglutiné
- confirmer la présence d'au moins une STEC d'un sérogroupe identifié par l'apparition d'au moins un halo d'une première couleur et d'au moins un halo d'une deuxième couleur autour de la même colonie ou d'au moins un halo résultant du mélange d'une première et d'une deuxième couleur autour de ladite colonie.

L'avantage de ce mode de réalisation est qu'il permet une discrimination des différents groupes de bactéries par un halo de couleur différent. Il est ainsi possible de distinguer visuellement
- un groupe de microorganismes sécrétant la toxine STX1 et/ou STX2
- un groupe de microorganismes choisis par O26, O45, O80, O91, O103, O104, O111, O113, O121, O128ab, O145, O146, O157, O174
- un groupe de microorganismes choisis parmi O26, O45, O80, O91, O103, O104, O111, O113, O121, O128ab, O145, O146, O157, O174 et sécrétant la toxine STX1 et/ou STX2.

Ainsi, en choisissant les nanoparticules de façons adéquates pour la formation du conjugué agglutinant, il est possible d'obtenir des halos de différentes couleurs et ainsi de discriminer différents groupes de microorganismes cibles.

Selon un autre mode de réalisation, la présente invention concerne une méthode de détection et de confirmation d'au moins une *Escherichia Coli entérohémorragique (EHEC)* susceptible d'être présente dans un échantillon comprenant des entérobactéries, comprenant les étapes suivantes :
- Effectuer une lyse de l'échantillon permettant la lyse des EHEC afin d'obtenir une solution comprenant leur acides nucléiques
- Mettre en contact la solution d'acides nucléiques avec des amorces permettant d'amplifier au moins le gène ou le fragment de gène stx1 et/ou stx2 et eae,
- si au moins un des gènes ou fragments des gènes stx1 et/ou stx2 et au moins un gène ou fragment de gène eae sont amplifiés, une partie de l'échantillon est déposée sur un milieu gélosé comprenant :
   ∘ au moins un inducteur de toxine
   ∘ au moins un conjugué agglutinant formé par au moins un partenaire de liaison spécifique de STX1 couplés à une nanoparticule d'une première nature produisant un halo d'une première couleur lorsqu'il est agglutiné
   ∘ et/ou au moins un conjugué agglutinant formé par au moins un partenaire de liaison spécifique de STX2 couplés à une nanoparticule d'une première nature produisant un halo d'une première couleur lorsqu'il est agglutiné
   ∘ Et au moins un conjugué agglutinant formé par au moins un partenaire de liaison spécifique de eae couplés à une nanoparticule d'une deuxième nature produisant un halo d'une deuxième couleur lorsqu'il est agglutiné
- confirmer la présence d'au moins une EHEC par l'apparition sur le milieu gélosé
   - d'au moins un halo d'une première couleur
   - et d'au moins un halo d'une deuxième couleur
   - ou d'au moins un halo résultant du mélange d'une première et d'une troisième couleur autour de ladite souche EHEC.

L'avantage de la méthode supra est qu'elle permet de discriminer dans un même échantillon les bactéries STEC productrices de shiga toxine STX1 et/ou STX2 des bactéries EHEC exprimant la protéine EAE.

### DESCRIPTION DES FIGURES

La figure 1 est une photo d'un milieu de culture comprenant des anticorps anti-STX1 et anti-STX2 sur lequel a été ensemencé un échantillon « viande hachée » contaminé avec une souche O80 portant les gènes eae stx2.
La figure 2 est une photo d'un milieu de culture comprenant des anticorps anti-STX1 et anti-STX2 sur lequel a été ensemencé un échantillon « carpaccio » contaminé avec une souche 045 portant les gènes eae stx1.

### DESCRIPTION DETAILLEE DE L'INVENTION

Certains termes et expressions utilisés dans le cadre de l'invention sont détaillés ci-après.

Un premier objet de l'invention concerne une méthode de détection et de confirmation d'au moins une *Escherichia Coli* productrice de shigatoxine (STEC) susceptible d'être présente dans un échantillon comprenant des entérobactéries, comprenant les étapes suivantes :
- Effectuer une lyse de l'échantillon permettant la lyse des STEC afin d'obtenir une solution comprenant leur acides nucléiques
- Mettre en contact la solution d'acides nucléiques avec des amorces permettant d'amplifier au moins le gène ou le fragment de gène stx1 et/ou stx2
- Si au moins un des gènes ou fragments des gènes stx1 et/ou stx2 est détecté, une partie de l'échantillon est déposé sur un milieu réactionnel gélosé comprenant
   - au moins un inducteur de toxine,
   - au moins un conjugué agglutinant formé par au moins un partenaire de liaison spécifique de la protéine STX1 et/ou au moins un partenaire de liaison spécifique de la protéine STX2, couplé à une nanoparticule.
- détecter et confirmer la présence d'au moins une STEC par l'apparition d'un halo sur la gélose autour de ladite STEC.

La méthode selon la présente invention présente l'avantage de confirmer la présence ou non d'une STEC directement sur le milieu réactionnel.

Par « détection », on entend la détection à l'oeil nu ou à l'aide d'un appareil optique de l'existence d'une croissance des micro-organismes cibles de préférence des bactéries cibles. Lorsque le milieu de culture à partir duquel on souhaite détecter les micro-organismes cibles comprend un substrat chromogène ou fluorogène, la détection peut être effectuée à l'aide d'un appareil optique pour les substrats fluorogènes, ou à l'oeil nu ou à l'aide d'un appareil optique pour les substrats chromogènes. Le milieu de culture comprenant un conjugué agglutinant, la détection peut se faire à l'oeil nu ou à l'aide d'un appareil optique en observant l'apparition d'un halo du fait de l'agglutination du conjugué autour du micro-organisme cible.

Par « confirmation », on entend le fait de confirmer par une seconde méthode un résultat présomptif positif obtenu par une première méthode de détection. Les deux méthodes peuvent être différentes ou non. Les technologies utilisées telles que la biologie moléculaire, l'immunologie, peuvent être également différentes ou non.

Par « isolement » on entend le fait d'obtenir des colonies différentes espacées les unes des autres.

L'échantillon peut être de diverses origines, par exemple d'origine alimentaire, environnementale, vétérinaire ou clinique. D'une manière générale, le terme « échantillon » se réfère à une partie ou à une quantité (plus particulièrement une petite partie ou une petite quantité) prélevées à partir d'une ou plusieurs entités aux fins d'analyse. Cet échantillon peut éventuellement avoir subi un traitement préalable, impliquant par exemple des étapes de mélange, de dilution ou encore de broyage, en particulier si l'entité de départ est à l'état solide.

L'échantillon peut subir une étape d'enrichissement pour faire croître les microorganismes et notamment les STEC. L'échantillon peut également subir une étape d'immuno-sélection afin de sélectionner au moins une souche STEC.

L'échantillon analysé est, en général, susceptible de - ou suspecté de - contenir au moins une *E. Coli* productrice de shigatoxine ou une *E. coli* entérohémorragique qu'il convient de détecter à des fins sanitaires. Les *E. coli* entérohémorragiques sont des souches représentant un sous-groupe des *E. coli* productrices de Shiga toxines STX, qui ont le gène eae et provoquent un syndrome hémolytique et urémique. La possession de ces deux facteurs de virulence en simultané rend ce pathovar très virulent pour l'Homme. Il s'agit notamment des sérogroupes O26, O45, O80, O91, O103, O104, O111, O113, O121, O128ab, O145, O146, O157, O174. Ainsi, la présente méthode permet de détecter des microorganismes cibles qui peuvent être le groupe des STEC, le groupe des EHEC typiques portant les gènes stx et eae, ou encore le groupe des EHEC atypiques portant le gène stx et aggR par exemple.

Selon un mode de réalisation de la présente invention, une étape d'incubation ou d'enrichissement est effectuée préalablement à la lyse de l'échantillon. Cette étape d'enrichissement requiert non seulement un milieu de culture *ad hoc* mais également une incubation de l'ensemble formé au moins par l'échantillon biologique et le milieu de culture à une température optimale pour permettre la croissance du/des micro-organisme(s) cible(s). L'incubation s'effectue, en général, à une température allant de 25 à 45°C durant un laps de temps prédéterminé (par exemple de 6h à 48h). Cette phase d'enrichissement requiert l'utilisation de milieux de culture, sélectifs ou non (en fonction du but recherché), qui ont pour but de promouvoir la croissance des micro-organismes cibles dans les échantillons, tout en limitant la croissance des flores non cibles. Les milieux de culture sont fréquemment utilisés dans des conteneurs de type sac en plastique stérile, dans lesquels ils sont mis en contact avec les échantillons alimentaires, cliniques ou environnementaux, aux fins de remise en suspension et enrichissement des micro-organismes recherchés. Tel que mentionné supra, cette phase d'enrichissement peut être nécessaire notamment afin de révéler la présence d'au moins un micro-organisme cible dans une quantité d'échantillon très variable et éventuellement très grande, par exemple de 25 grammes (g) à 375 g dilués dans un volume de milieu de culture compris entre 225 et 3375 millilitres (mL). A l'issue de cette étape d'enrichissement, un aliquote (généralement d'un volume compris entre 5 microlitres (µL) et 5 mL) est traditionnellement prélevé pour mettre en oeuvre l'étape de détection des micro-organismes cibles.

Selon la présente invention, l'échantillon en microorganismes cibles subit une lyse. En effet, afin d'effectuer une amplification, il est nécessaire d'extraire l'ADN des bactéries. On utilisera par exemple, le kit de lyse GENE-UP^{®}. Tout autre méthode de lyse connue de l'homme du métier est envisageable.

Il peut être souhaitable d'effectuer une immuno-sélection d'au moins une STEC avant l'étape de lyse. On utilisera par exemple le kit VIDAS^{®} ESPT et l'instrument VIDAS^{®} de la demanderesse. L'immuno-sélection permet d'augmenter la proportion des bactéries d'intérêt par rapport à la flore annexe. Elle est avantageuse lorsque l'échantillon polymicrobien possède une quantité de flore importante comme les produits laitiers. La présence en faible quantité d'une STEC ou d'une EHEC est alors difficilement détectable. Il y a un risque de faux négatif. Selon la présente invention, une étape d'amplification d'au moins un des gènes ou fragments des gènes stx1 et/ou stx2 est effectuée. Il est également possible d'amplifier les sous-types de stx1 ou stx2 tels que stx2a ou encore stx2d.

L'amplification peut être réalisée par toute méthode de biologie moléculaire connue de l'homme du métier. On peut citer les méthodes d'amplification telles que toutes les méthodes de PCR ou encore les méthodes isothermales.

A l'issue de la détection par PCR, si un échantillon est présomptif positif, il est nécessaire d'effectuer une confirmation. Avant l'ensemencement sur un milieu de réactionnel, il peut être souhaitable d'effectuer une immuno-sélection des microorganismes. L'immuno-sélection est avantageuse lorsque l'échantillon contient une flore annexe importante. En effet, cela permet de réduire la diversité de bactéries qui seront visibles sur boite et d'en simplifier la discrimination entre les STEC des sérogroupes ciblés et les non-STEC.

Selon la présente invention, la confirmation est effectuée à partir d'un aliquot de l'échantillon ou de l'enrichissement ou d'un échantillon ayant subi une étape d'immuno-sélection d'au moins une STEC d'un sérogroupe ciblé. La confirmation est réalisée sur un milieu réactionnel comprenant au moins un inducteur de toxine et au moins un partenaire de liaison spécifique d'un composant d'une *E. coli* productrices de shigatoxines ou d'un composant issu de cette dernière, couplé à au moins une nanoparticule pour former au moins un conjugué agglutinant. Avantageusement, le milieu de culture comprend un partenaire de liaison spécifique de STX1 et/ou un partenaire de liaison spécifique de STX2. Il est également possible d'avoir un partenaire de liaison spécifique de certains sous-types de STX1 ou STX2 tel que STX2a ou STX2d.

L'ensemencement est réalisé selon des techniques classiques de microbiologie. Dans un mode préférentiel de l'invention, l'ensemencement se fait par un épuisement de l'échantillon polymicrobien sur le milieu de culture. Les techniques d'ensemencement par épuisement sont bien connues par l'homme du métier. Il peut s'agir d'un ensemencement en quadrants. La méthode des quadrants consiste à diviser une boîte de Pétri en deux, puis de diviser de nouveau par deux une moitié afin d'obtenir 3 quadrants de 50 %, 25 % et 25 %. Sur le plus grand quadrant une petite quantité d'inoculum est posée puis étalée. La boîte est ensuite tournée d'un quart afin d'étaler les bactéries sur un quadrant plus petit, puis de nouveau tournée d'un quart afin d'ensemencer le dernier petit quadrant. Il peut également s'agir également d'un épuisement par stries multiples qui consiste à étaler l'inoculum vers le bas en stries serrées puis à nouveaux d'autres stries partant des bords des précédentes bords à bords.

Suite à l'ensemencement, le milieu réactionnel est incubé dans des conditions appropriées connues de l'homme de l'art.

Selon la présente invention, le milieu réactionnel est un milieu réactionnel gélifié comprenant au moins un inducteur de toxine et au moins un partenaire de liaison spécifique de STX1 et/ou au moins un partenaire de liaison spécifique de STX2, couplé à une nanoparticule.

Par « milieu réactionnel », on entend un milieu comprenant tous les éléments nécessaires à l'expression d'un métabolisme, à la survie et/ou la croissance des micro-organismes. Ce milieu réactionnel peut soit être un milieu de culture microbiologique, soit être un milieu de révélation microbiologique. Dans ce dernier cas, la culture des micro-organismes peut être effectuée préalablement dans un autre milieu. Le milieu réactionnel peut également être mis en contact avec un milieu de culture gélosé. Il peut être placé sous ou sur le milieu de culture qui permet la croissance des micro-organismes cibles. Le milieu réactionnel peut être ajouté après l'incubation du milieu de culture. Le milieu réactionnel est gélifié. Il se présente sous forme solide ou semi-solide. L'agar est l'agent gélifiant traditionnel utilisé en microbiologie pour la culture des micro-organismes, mais il est possible d'utiliser d'autres agents gélifiants comme par exemple, la gélatine, l'agarose ainsi que d'autres gélifiants naturels ou artificiels. Le conjugué est capable de former un réseau avec le composant cible dans un milieu gélifié. Ce réseau est visuellement détectable par la formation d'un halo.

Un certain nombre de préparations sont disponibles dans le commerce, comme par exemple l'agar Columbia, la gélose Trypcase-soja, la gélose Mac Conkey, la gélose Mueller Hinton ou plus généralement celles décrites dans le Handbook of Microbiological Media. Ces milieux peuvent servir de base au milieu réactionnel selon l'invention. Le milieu réactionnel peut en outre comprendre d'éventuels additifs comme par exemple des acides aminés, des peptones, un ou plusieurs facteurs de croissance, des hydrates de carbone, des nucléotides, des minéraux, des vitamines, un ou plusieurs agents sélectifs, des inducteurs, des inducteurs de toxines, des tampons etc. Par « agent sélectif », on entend tout composé susceptible d'empêcher ou de ralentir la croissance d'un micro-organisme dit « non-cible », à savoir autre que le ou les micro-organisme(s) cible(s). Le terme « inducteur » se réfère à un composé capable d'induire l'expression d'un composé, telle une enzyme, une toxine, qui normalement resterait inexprimé. Ledit milieu réactionnel peut également comprendre un colorant. A titre indicatif, on peut citer comme colorant le bleu d'Evans, du rouge neutre, du sang de mouton, du sang de cheval, un opacifiant tel que l'oxyde de Titane, de la nitroaniline, du vert malachite, du vert brillant. Lorsque le milieu réactionnel comprend en outre un substrat enzymatique spécifique d'une activité enzymatique d'au moins un micro-organisme cible, on utilise de préférence un substrat chromogène et/ou fluorogène. Par «substrat chromogène et/ou fluorogène », on entend un substrat permettant la détection d'une activité enzymatique ou métabolique des micro-organismes cibles/recherchés grâce à un signal détectable. Le milieu réactionnel peut comporter en sus un indicateur de pH, sensible à la variation de pH induite par la consommation du substrat et révélant le métabolisme des micro-organismes cibles. Ledit indicateur de pH peut être un chromophore ou un fluorophore. On citera, comme exemple de chromophore, le bromocresol pourpre, le bleu de bromothymol, le rouge neutre, le bleu d'aniline, le bleu de bromocresol.

L'homme du métier peut également utiliser une boîte de Pétri divisée en segment, telle qu'une bi-boîte, ou une tri-boîte, permettant de comparer aisément plusieurs milieux, comprenant différents substrats ou différents mélanges sélectifs, sur lesquels un même échantillon biologique aura été déposé.

Selon la présente invention, le milieu réactionnel comprend un partenaire de liaison spécifique d'un composant d'un micro-organisme cible ou d'un composant issu dudit micro-organisme, couplé à une nanoparticule. Le partenaire de liaison spécifique est choisi parmi les anticorps, tous types de fragments Fab, les protéines recombinantes, les phages, les protéines de phage, les oligonucléotides, les aptamères, les affimères ou tout autre ligand ou anti-ligand bien connu de l'homme du métier. Préférentiellement le partenaire de liaison est un anticorps ou une protéine de phage. Préférentiellement, l'anticorps est un anticorps monoclonal ou un fragment d'anticorps monoclonal. Le partenaire de liaison est spécifique d'un composant d'un micro-organisme cible. Le composant du micro-organisme cible est un composant relargué par ledit micro-organisme. Le composant peut ainsi être un élément issu de la surface de la bactérie tel qu'une protéine, un Lipopolysaccharide (LPS), un flagelle. Il peut également s'agir d'un élément interne à la bactérie tel que l'ARN, une protéine intra-cellulaire qui pourra être détecté lors de la mort d'une partie de la colonie bactérienne au cours de la croissance de celle-ci. Dans un mode avantageux de l'invention, le partenaire de liaison peut aussi être spécifique d'un composant issu dudit micro-organisme. Ce composant peut être une molécule d'intérêt produite par le micro-organisme cible telle qu'une protéine, un antibiotique, une molécule de résistance à des agents antimicrobiens, des enzymes type protéases, des lipases ou glusidases. Préférentiellement, le milieu comprend au moins un partenaire de liaison spécifique de la protéine STX1 et/ou au moins un partenaire de liaison spécifique de la protéine STX2. Préférentiellement, le milieu comprend au moins un partenaire de liaison spécifique de STX2a et/ou STX2d.

Selon la présente invention le partenaire de liaison est couplé à une nanoparticule. Ce complexe final est appelé conjugué. Dans un même milieu réactionnel, il est possible d'avoir des conjugués ayant des partenaires de liaison de nature différente couplés à des nanoparticules elles même de nature différente. Le terme « nanoparticule » désigne les particules de l'ordre de grandeur du nanomètre. Les nanoparticules peuvent être choisies parmi l'or, le fer, l'argent, le cuivre, le carbone, le latex, le silicium, l'aluminium. Préférentiellement, les nanoparticules sont des nanoparticules colloïdales choisies pour leur propriété optique à savoir leur capacité à se distinguer lorsqu' un réseau se forme. De façon encore plus préférentielle, la nanoparticule est choisie parmi l'or, l'argent, le cuivre. Ainsi, lorsque les nanoparticules sont en or, elles changent de couleur, passant par exemple du rouge au gris lorsqu'elles forment un réseau. Lorsqu'elles ne sont pas agrégées, La longueur d'onde de la lumière absorbée est dans le rouge autour de 530nm. Lorsqu'elles sont agrégées, la longueur d'onde absorbée change du rouge au bleu/gris autour de 600 à 700nm. Dans un mode de réalisation particulier, il est possible d'utiliser ensemble plusieurs nanoparticules de couleurs différentes. Les réseaux ainsi formés permettent la distinction de plusieurs groupes de micro-organismes cibles. Préférentiellement, les nanoparticules ont une taille comprise entre 10 et 200nm. Préférentiellement les nanoparticules ont une taille comprise entre 20 et 90nm, permettant une meilleure mobilité des conjugués dans le milieu réactionnel.

Avantageusement les nanoparticules permettent de diminuer la quantité nécessaire de partenaires de liaison pour la formation d'une agglutination. Ainsi, la concentration nécessaire en partenaires de liaison pour la réalisation d'un milieu réactionnel selon l'invention nécessite 100 à 1000 fois moins de partenaires de liaison qu'un milieu sans nanoparticule. Préférentiellement, la quantité nécessaire en partenaires de liaison correspond à la quantité nécessaire pour recouvrir au minimum la moitié de la surface de la nanoparticule, et encore plus préférentiellement pour recouvrir entre le tiers et la moitié de la surface de la nanoparticule. Cette proportion permet au conjugué agglutinant de former un réseau dans le milieu réactionnel gélifié. Avantageusement, les nanoparticules peuvent permettre de visualiser une agglutination autour d'une colonie bactérienne dont la taille ne permet pas encore d'être visible à l'oeil nu. La détection peut ainsi être plus précoce. Avantageusement, les nanoparticules peuvent permettre de visualiser une agglutination autour d'une colonie bactérienne dont l'aspect translucide ne permet pas la détection par un automate de lecture. La détection peut ainsi être facilité.

Dans un mode de réalisation particulier, le partenaire de liaison est un anticorps présent à une quantité permettant de recouvrir au minimum la moitié de la surface de la nanoparticule et préférentiellement au minimum le tiers de la surface de la nanoparticule. Préférentiellement, dans ces variantes la nanoparticule est une nanoparticule d'or de taille comprise entre 20 et 90nm et à une concentration comprise entre 10¹⁰ et 10¹² nanoparticules/ml de milieu réactionnel. Le couplage de la nanoparticule au partenaire de liaison peut se faire soit par une fixation directe soit par une fixation indirecte. Par fixation directe, on entend la fixation par adsorption, soit par liaison covalente. Par fixation indirecte, on entend une fixation par l'interaction de ligands/anti-ligands comme par exemple la biotine/strepatividine ou d'autres couples bien connus de l'homme du métier. En fonction du type de liaison choisie, l'homme du métier adaptera les conditions physico-chimiques du milieu réactionnel et notamment son pH.

Selon la présente invention, le conjugué est agglutinant c'est-à-dire qu'il provoque la formation d'un réseau d'agglutination en présence d'un composant d'un micro-organisme cible ou d'un composant issu dudit micro-organisme. Le composant étant multiépitope, plusieurs conjugués vont se fixer à ce composant et former une agglutination. Par agglutination, on entend le résultat d'une interaction entre au moins un composant d'un micro-organisme cible ou au moins un composant issu dudit micro-organisme avec des partenaires de liaison couplés à une nanoparticule. Les réactions d'agglutination comprennent des réactions immunologiques, telles des réactions antigène-anticorps ou plus généralement des interactions spécifiques entre deux molécules. Par cette interaction, composants et conjugués s'agrègent, adhèrent entre eux et forment un réseau dans le milieu réactionnel. En pratique, plusieurs paramètres influencent la capacité du conjugué à être agglutinant dans un milieu gélifié comme principalement :
- la porosité du milieu gélifié
- la taille des nanoparticules
- la quantité de partenaires de liaison
- la quantité de nanoparticules

Il conviendra donc d'adapter ces paramètres afin de permettre une agglutination satisfaisante permettant sa détection. Avantageusement, le réseau formé par ladite réaction spécifique est alors détecté soit visuellement, soit de façon automatique grâce à un système optique. La colonie du micro-organisme cible est ainsi repérée. De préférence, le réseau forme un halo dans le milieu réactionnel gélifié détectable soit visuellement soit grâce à un système optique. Le réseau ou le halo circonscrit ainsi la colonie qui peut alors être avantageusement différenciée et/ou identifiée au sein d'une population.

Selon la présente invention, le milieu réactionnel comprend un inducteur de toxine. L'inducteur de toxine provoque un stress chez la bactérie qui va déclencher le cycle lytique des prophages dans la bactérie et donc stimuler la production de toxines qui seront libérées. On citera les shigatoxines sécrétées par les souches STEC. Il existe deux grands types de shiga-toxines : STX1 et STX2 qui elles-mêmes présentent de nombreux variants. A ce jour, STX1 a 4 sous types STX1a, STX1c, STX1d, STX1e, et STX2 en a 12: STX2a, STX2b STX2c, STX2d, STX2e, STX2f, STX2g, STX2h, STX2i, STX2j, STX2K, STX2l. De manière avantageuse, l'inducteur de toxine est choisi parmi les antibiotiques ou un stress physico-chimique. On citera, en tant qu'antibiotique, trimethoprime, sulfamethoxazole, norfloxacine, azithromycine, gemtamicine, polymyxine B, chloramphénicol, streptomycine, chlortetracycline, oxytetracyline, tylosine, mitomycine C, carbodox, oliquindox, rifampincine, imipenème, ciprofloxacine, cotrimoxazole, pénicilline G, linlomycine. Dans un mode de réalisation préféré, le milieu selon l'invention comprend de la ciprofloxacine à une concentration comprise entre 0,005 et 0,030 mg/l. Dans un autre mode de réalisation préféré le milieu selon l'invention comprend de la mitomycine C à une concentration comprise entre 0.10 et 0.50 mg/l.

L'inducteur de toxine peut également être un stress physico-chimique réalisé, par exemple, par l'ajout de sel ou d'EDTA ou par une modification du pH ou encore par un stress UV. Le stress peut également être, par exemple, induit par l'ajout de noradrénaline.

Avantageusement, le milieu réactionnel peut être fabriqué extemporanément. Cela permet l'utilisation unitaire d'un milieu réactionnel, et d'augmenter la stabilité de celui-ci.

Le procédé d'obtention d'un milieu réactionnel comprend les étapes de mise en contact d'un conjugué agglutinant avec un milieu gélifiant pour former le milieu réactionnel. La préparation du conjugué s'effectue par le couplage du partenaire de liaison avec la nanoparticule. Ces méthodes de couplage sont bien connues de l'homme du métier (Nicholas G. Weich et al, 2017). Le conjugué est ensuite ajouté au milieu gélifié mis en surfusion. L'ensemble est ensuite homogénéisé et coulé dans la boîte de Pétri.

Selon la présente invention, la présence d'au moins une STEC est détectée et confirmée par l'apparition d'un halo sur la gélose autour de la dite STEC.

Dans ce mode de réalisation il peut également être intéressant de détecter par biologie moléculaire les sous-types de STX1 et/ou STX2 détectés sur la gélose tel que STX2a et/ou STX2d.

Un autre objet de l'invention concerne une méthode de détection et de confirmation d'au moins une *E. Coli* productrice de shigatoxine comprenant les étapes suivantes :
- Effectuer une lyse de l'échantillon permettant la lyse des STEC afin d'obtenir une solution comprenant leurs acides nucléiques
- Mettre en contact la solution d'acides nucléiques avec des amorces permettant d'amplifier au moins le gène ou le fragment de gène stx1 et/ou stx2
- Si au moins un des gènes ou fragments des gènes stx1 et/ou stx2 est amplifié, la solution d'acides nucléiques est mise en contact avec des amorces permettant d'amplifier au moins un gène ou un fragment de gène d'un sérogroupe choisi parmi O26, O80, O45, O91, O103, 0104, O111, O121, O128ab, O145, O146, O113, O157, O174.
- si au moins un des gènes ou fragments des gènes stx1 et/ou stx2 et au moins un sérogroupe choisi parmi O26, O80, O45, O91, O103, O104, O111, O121, O128ab, O145, O146, O113, O157, O174, sont amplifiés, une partie de l'échantillon est déposé sur un milieu réactionnel comprenant
   ∘ au moins un inducteur de toxine et
   ∘ au moins un conjugué agglutinant formé par au moins un partenaire de liaison spécifique de la protéine STX1 et/ou au moins un partenaire de liaison spécifique de la protéine STX2, couplé(s) à une nanoparticule.
- détecter et confirmer la présence d'au moins une STEC par l'apparition d'un halo sur la gélose autour de ladite STEC.

L'avantage de cette méthode est qu'elle permet de détecter et confirmer la présence de STEC. De plus, l'approche sérogroupale permet de renforcer la présomption qu'il s'agit d'une STEC d'un sérogroupe cible. Dans ce mode de de réalisation, le sérogroupe peut être confirmé par biologie moléculaire à partir de la colonie isolée. Le sérogroupe peut également être confirmé par test d'agglutination de particules de latex tel que SLIDEX^{®} *E. coli* O26 ou *E. coli* O80 etc...

Un autre objet de l'invention concerne une méthode de détection et de confirmation d'au moins une *E. Coli* productrice de shigatoxine susceptible d'être présente dans un échantillon comprenant des entérobactéries, comprenant les étapes suivantes :
- Effectuer une lyse de l'échantillon permettant la lyse des STEC afin d'obtenir une solution comprenant leur acides nucléiques
- Mettre en contact la solution d'acides nucléiques avec des amorces permettant d'amplifier au moins le gène ou le fragment de gène stx1 et/ou stx2
- si au moins un des gènes ou fragments des gènes stx1 et/ou stx2 est amplifié et au moins un gène ou le fragment de gène d'au moins un sérogroupe choisi parmi O26, O45, O80, O91, O103, O104, O111, O113, O121, O128ab, O145, O146, O157, O174 sont amplifiés, une partie de l'échantillon est déposé sur un milieu réactionnel comprenant :
   ∘ au moins un inducteur de toxine et
   ∘ au moins un conjugué agglutinant formé par au moins un partenaire de liaison spécifique de STX1 couplé à une nanoparticule d'une première nature produisant un halo d'une première couleur lorsqu'il est agglutiné
   ∘ et/ou au moins un conjugué agglutinant formé par au moins un partenaire de liaison spécifique de STX2 couplé à une nanoparticule d'une première nature produisant un halo d'une première couleur lorsqu'il est agglutiné
   ∘ et au moins un conjugué agglutinant formé par au moins un partenaire de liaison spécifique dudit sérotype identifié par amplification couplé à une nanoparticule d'une deuxième nature produisant un halo d'une deuxième couleur lorsqu'il est agglutiné
- confirmer la présence d'au moins une STEC d'un sérogroupe identifié par l'apparition d'au moins un halo d'une première couleur et d'au moins un halo d'une deuxième couleur ou d'au moins un halo résultant du mélange d'une première et d'une deuxième couleur autour de ladite souche

L'avantage de la méthode sérogroupale supra est qu'elle permet de détecter et confirmer la présence d'une STEC d'un sérogroupe cible. L'avantage de ce mode de réalisation est qu'il permet une discrimination des différents groupes de bactéries par un halo de couleur différents. Il est ainsi possible de distinguer visuellement
- un groupe de microorganismes sécrétant la toxine STX1 et/ou STX2
- un groupe de microorganismes choisis par O26, O45, O80, O91, O103, O104, O111, O113, O121, O128ab, O145, O146, O157, O174
- un groupe de microorganismes choisis parmi O26, O45, O80, O91, O103, O104, O111, O113, O121, O128ab, O145, O146, O157, O174 et sécrétant la toxine STX1 et/ou STX2.

Ainsi, en choisissant les nanoparticules de façons adéquates pour la formation du conjugué agglutinant, il est possible d'obtenir des halos de différentes couleurs et ainsi de discriminer différents groupes de microorganismes cibles. Il est ainsi possible d'envisager un autre mode de réalisation de la méthode comprenant les étapes suivantes :
- Effectuer une lyse de l'échantillon permettant la lyse des STEC afin d'obtenir une solution comprenant leurs acides nucléiques
- Mettre en contact la solution d'acides nucléiques avec des amorces permettant d'amplifier au moins le gène ou le fragment de gène stx1 et/ou stx2
- Si au moins un des gènes ou fragments des gènes stx1 et/ou stx2 est amplifié et au moins un gène ou le fragment de gène d'au moins un sérogroupe choisi parmi O26, O45, O80, O91, O103, O104, O111, O113, O121, O128ab, O145, O146, O157, O174 sont amplifiés, une partie de l'échantillon est déposé sur un milieu réactionnel comprenant :
   ∘ au moins un inducteur de toxine et
   ∘ au moins un conjugué agglutinant formé par au moins un partenaire de liaison spécifique de STX1 couplé à une nanoparticule d'une première nature produisant un halo d'une première couleur lorsqu'il est agglutiné
   ∘ et/ou au moins un conjugué agglutinant formé par au moins un partenaire de liaison spécifique de STX2 couplé à une nanoparticule d'une deuxième nature produisant un halo d'une deuxième couleur lorsqu'il est agglutiné
   ∘ et au moins un conjugué agglutinant formé par au moins un partenaire de liaison spécifique dudit sérogroupe identifié par amplification couplé à une nanoparticule d'une troisième nature produisant un halo d'une troisième couleur lorsqu'il est agglutiné
- confirmer la présence ou non d'au moins une STEC d'un sérogroupe identifié par l'apparition sur le milieu gélosé :
   - d'au moins un halo d'une première couleur ou deuxième couleur
   - et d'au moins un halo d'une troisième couleur
   - ou d'au moins un halo résultant du mélange d'une première et/ou d'une deuxième couleur et d'une troisième couleur autour de ladite souche

Ce mode de réalisation permet une discrimination des différents groupes de bactéries par un halo de couleur différents. Il est ainsi possible de distinguer visuellement
- un groupe de microorganismes sécrétant la toxine STX1
- un groupe de microorganismes sécrétant la toxine STX2
- un groupe de microorganismes choisis parmi O26, O80, O45, O91, O103, O104, O111, O121, O128ab, O145, O146, O113, O157, O174.
- un groupe de microorganismes choisis parmi O26, O80, O45, O91, O103, O104, O111, O121, O128ab, O145, O146, O113, O157, O174 et sécrétant la toxine STX1 et/ou STX2.

Selon la présente invention, il est également possible de détecter et de confirmer la présence d'une *Escherichia Coli entérohémorragique (EHEC).* Une EHEC est une STEC ayant le gène eae. Ainsi, un autre objet de l'invention concerne une méthode de détection et de confirmation d'au moins une *Escherichia Coli entérohémorragique (EHEC)* susceptible d'être présente dans un échantillon comprenant des entérobactéries, comprenant les étapes suivantes :
- incuber l'échantillon dans un milieu d'enrichissement permettant la croissance des EHEC
- Effectuer une lyse de l'échantillon permettant la lyse des EHEC afin d'obtenir une solution comprenant leurs acides nucléiques
- Mettre en contact la solution d'acides nucléiques avec des amorces permettant d'amplifier au moins le gène ou le fragment de gène stx1 et/ou stx2 et eae,
- si au moins un des gènes ou fragments des gènes stx1 et/ou stx2 et au moins un gène ou fragment de gène eae sont amplifiés, déposer une partie de l'échantillon enrichi sur un milieu gélosé comprenant :
   ∘ au moins un inducteur de toxine
   ∘ au moins un conjugué agglutinant formé par au moins un partenaire de liaison spécifique de STX1 couplés à une nanoparticule d'une première nature produisant un halo d'une première couleur lorsqu'il est agglutiné
   ∘ et/ou au moins un conjugué agglutinant formé par au moins un partenaire de liaison spécifique de STX2 couplés à une nanoparticule d'une première nature produisant un halo d'une première couleur lorsqu'il est agglutiné
   ∘ Et au moins un conjugué agglutinant formé par au moins un partenaire de liaison spécifique de eae couplés à une nanoparticule d'une deuxième nature produisant un halo d'une deuxième couleur lorsqu'il est agglutiné
- confirmer la présence d'au moins une EHEC par l'apparition sur le milieu gélosé
   - d'au moins un halo d'une première couleur
   - et d'au moins un halo d'une deuxième couleur
   - ou d'au moins un halo résultant du mélange d'une première et d'une troisième couleur autour de ladite souche EHEC

L'avantage de la méthode supra est qu'elle permet discriminer dans un même échantillon les bactéries STEC productrices de shiga toxine STX1 et/ou STX2 des bactéries EHEC exprimant la protéine EAE.

Un autre objet de l'invention concerne une méthode de détection et de confirmation d'au moins une *Escherichia Coli entérohémorragique (EHEC)* susceptible d'être présente dans un échantillon comprenant des entérobactéries, comprenant les étapes suivantes :
- incuber l'échantillon dans un milieu d'enrichissement permettant la croissance des EHEC
- Effectuer une lyse de l'échantillon enrichi permettant la lyse des EHEC afin d'obtenir une solution comprenant leur acides nucléiques
- Mettre en contact la solution d'acides nucléiques avec des amorces permettant d'amplifier au moins le gène ou le fragment de gène stx1 et/ou stx2 et eae,
- Si au moins un des gènes ou fragments des gènes stx1 et/ou stx2 et au moins un des gènes ou fragments du gène eae sont amplifiés, déposer une partie de l'échantillon enrichi sur un milieu gélosé comprenant :
   ∘ au moins un inducteur de toxine
   ∘ au moins un conjugué agglutinant formé par au moins un partenaire de liaison spécifique de STX1 couplés à une nanoparticule d'une première nature produisant un halo d'une première couleur lorsqu'il est agglutiné
   ∘ et/ou au moins un conjugué agglutinant formé par au moins un partenaire de liaison spécifique de STX2 couplés à une nanoparticule d'une deuxième nature produisant un halo d'une deuxième couleur lorsqu'il est agglutiné
   ∘ Et au moins un conjugué agglutinant formé par au moins un partenaire de liaison spécifique de EAE couplés à une nanoparticule d'une troisième nature produisant un halo d'une troisième couleur lorsqu'il est agglutiné
- confirmer ou non la présence d'au moins une EHEC par l'apparition sur le milieu gélosé :
   - d'au moins un halo d'une première couleur ou deuxième couleur
   - et d'au moins un halo d'une troisième couleur
   - ou d'au moins un halo résultant du mélange d'une première et/ou d'une deuxième couleur et d'une troisième couleur autour de ladite souche EHEC

L'avantage de ce mode de réalisation est qu'il permet une discrimination des différents groupes de bactéries par un halo de couleur différents. Il est ainsi possible de distinguer visuellement
- un groupe de microorganismes sécrétant la toxine STX1
- un groupe de microorganismes sécrétant la toxine STX2
- un groupe de microorganismes exprimant la protéine EAE

Un autre objet de l'invention concerne une méthode de détection et de confirmation d'au moins une *Escherichia Coli* productrice de shigatoxine (STEC) et/ou d'*Escherichia Coli entérohémorragique (EHEC)* comprenant les étapes suivantes :
- Effectuer une lyse de l'échantillon permettant la lyse des STEC ou EHEC afin d'obtenir une solution comprenant leurs acides nucléiques
- Mettre en contact la solution d'acides nucléiques avec des amorces permettant d'amplifier au moins le gène ou le fragment de gène stx1 et/ou stx2 et eae ainsi qu' au moins un gène ou fragment de gène d'au moins un sérogroupe choisi parmi O26, O45, O80, O91, O103, O104, O111, O113, O121, O128ab, O145, O146, O157, O174
- si au moins un des gènes ou fragments des gènes stx1 et/ou stx2 et un gène ou fragment de gène d'eae sont amplifiés, une partie de l'échantillon est déposé sur un milieu réactionnel comprenant
   ∘ au moins un inducteur de toxine et
   ∘ au moins un conjugué agglutinant formé par au moins un partenaire de liaison spécifique de STX1 couplés à une nanoparticule d'une première nature produisant un halo d'une première couleur lorsqu'il est agglutiné
   ∘ et/ou au moins un conjugué agglutinant formé par au moins un partenaire de liaison spécifique de STX2 couplés à une nanoparticule d'une première nature produisant un halo d'une première couleur lorsqu'il est agglutiné
   ∘ Et au moins un conjugué agglutinant formé par au moins un partenaire de liaison spécifique de eae couplés à une nanoparticule d'une deuxième nature produisant un halo d'une deuxième couleur lorsqu'il est agglutiné
- confirmer la présence d'au moins une STEC par l'apparition sur le milieu gélosé
   - d'au moins un halo d'une première couleur
- confirmer la présence d'au moins une EHEC par l'apparition sur le milie ugélosé
   - d'au moins un halo d'une première couleur
   - et d'au moins un halo d'une deuxième couleur
   - ou d'au moins un halo résultant du mélange d'une première et d'une deuxième couleur autour de ladite souche

L'avantage de la méthode supra est qu'elle permet une approche sérogroupale dans laquelle La présomption d'une STEC et/ou d'une EHEC d'un sérogroupe type est renforcé.

Dans un autre mode de réalisation de la méthode, la solution d'acides nucléiques est mise en contact avec des amorces permettant d'amplifier le gène ou le fragment de gène aggR permettant de détecter également les *E. coli* enteroaggregative.

Un autre objet de l'invention concerne une méthode de détection et confirmation d'au moins une *Escherichia Coli* productrice de shigatoxine (STEC) susceptible d'être présente dans un échantillon comprenant des entérobactéries, comprenant les étapes suivantes :
- mettre en contact l'échantillon avec un milieu d'enrichissement
- mettre en contact l'échantillon avec des anticorps anti-STX1 et anti-STX2
- si la présence d'au moins une protéine STX1 et/ou une protéine STX2 est détectée, une partie de l'échantillon est déposé sur un milieu réactionnel gélosé comprenant
   ▪ au moins un inducteur de toxine,
   ▪ au moins un conjugué agglutinant formé par au moins un partenaire de liaison spécifique de la protéine STX1 et/ou au moins un partenaire de liaison spécifique de la protéine STX2, couplé(s) à une nanoparticule.
- Détecter et confirmer la présence d'au moins une STEC par l'apparition d'un halo sur la gélose autour de ladite STEC.

Préférentiellement, l'échantillon est mis en contact avec un inducteur de toxine pendant ou après enrichissement. L'inducteur de toxine provoque un stress chez la bactérie qui va déclencher le cycle lytique des prophages dans la bactérie et donc stimuler la production de toxines qui seront libérées. On citera les shigatoxines sécrétées par les souches STEC. Il existe deux grands types de shiga-toxines : STX1 et STX2 qui elles-mêmes présentent de nombreux variants. A ce jour, STX1 a 4 sous types STX1a, STX1c, STX1d, STX1e, et STX2 en a 12: STX2a, STX2b STX2c, STX2d, STX2e, STX2f, STX2g, STX2h, STX2i, STX2j, STX2K, STX2l. De manière avantageuse, l'inducteur de toxine est choisi parmi les antibiotiques ou un stress physico-chimique. On citera, en tant qu'antibiotique, trimethoprime, sulfamethoxazole, norfloxacine, azithromycine, gemtamicine, polymyxine B, chloramphénicol, streptomycine, chlortetracycline, oxytetracyline, tylosine, mitomycine C, carbodox, oliquindox, rifampincine, imipenème, ciprofloxacine, cotrimoxazole, pénicilline G, linlomycine. Dans un mode de réalisation préféré, le milieu selon l'invention comprend de la ciprofloxacine à une concentration comprise entre 0,005 et 0,030 mg/l. Dans un autre mode de réalisation préféré le milieu selon l'invention comprend de la mitomycine C à une concentration comprise entre 0.10 et 0.50 mg/l.

L'inducteur de toxine peut également être un stress physico-chimique réalisé, par exemple, par l'ajout de sel ou d'EDTA ou par une modification du pH ou encore par un stress UV. Le stress peut également être, par exemple, induit par l'ajout de noradrénaline.

Selon ce mode de réalisation de l'invention, l'échantillon est mis en contact avec des anticorps anti-STX1 et anti-STX2. On utilisera par exemple l'instrument VIDAS^{®} de la demanderesse. Selon un autre mode de réalisation, les anticorps spécifiques utilisés sont des anticorps anti-STX1a et/ou anti-STX1d. l'échantillon est ensuite déposé sur un milieu de culture tel que décrit supra.

La présente invention permet avantageusement de détecter et de confirmer la présence d'une souche STEC et/ou une souche EHEC par la formation d'un halo autour de ladite souche. Cette invention est particulièrement intéressante pour faciliter la détection et la confirmation de la présence des STEC ou des EHEC dans un échantillon polymicrobien. En effet, sans la présente invention qui permet de localiser la colonie d'intérêt, la méthode de référence ISO 16136 précise qu'il est nécessaire de tester jusqu'à 50 colonies par méthode moléculaire pour confirmer la présence d'un STEC. Au regard du nombre important de colonies sur la boite et la faible représentation du micro-organisme cible, la personne prélevant les colonies à des fins de confirmation, peut ne jamais prélever le micro-organisme cible. Ainsi la présente invention permet d'éviter les faux négatifs et permet un gain de temps dans l'exécution du contrôle microbiologique. Elle est particulièrement avantageuse pour des échantillons chargés en flore annexe, où la quantité importante de colonies sur les boîtes de Pétri peut entraîner un risque de faux négatifs.

### Exemples

### Exemple 1- Préparation des échantillons et d'un milieu de de culture

### - Préparation des échantillons contaminés de « viande hachée » :

Un échantillon de 25g de haché au boeuf avec protéines végétales (Réf Carrefour, lot 671579) a été artificiellement contaminé par équilibration avec une suspension bactérienne de la souche *E. coli* sérogroupe O80 possédant les marqueurs de virulence eae stx2 (Centre Hospitalier Robert Debré Paris REF H15-66,3). L'équilibration a été réalisée 48/72h à 2-8°C. Les taux d'inoculations pour les échantillons générés étaient respectivement de 1,5 et 3cfu/25g. Un contrôle négatif a également été réalisé : 25g de haché au boeuf avec protéines végétales qui n'a pas été contaminé artificiellement. Les échantillons artificiellement contaminés ont ensuite été dilués au 1/10 avec de l'eau peptonnée tamponnée (BPW Dilubag 5L ref AEB910305/2, bioMérieux) dans un sac d'enrichissement. Une homogénéisation a été réalisée au malaxeur à pâles (Seward Stomacher), puis les sacs ont été placés à l'étuve à 41,5°C pour 8-24h.

### - Préparation des échantillons contaminés de « carpaccio » :

Un échantillon de 25g de Carpaccio marinade au basilic (Charal, lot 71394005) a été artificiellement contaminé par équilibration avec une suspension bactérienne de la souche Escherichia coli serogroupe O45 ( ATCC BAA-2198) porteuse des gènes eae et stx1. L'équilibration a été réalisée 48/72h à 2-8°C. Le taux d'inoculation pour l'échantillon généré était de 1,1 cfu/25g. Un contrôle négatif a également été réalisé : 25g de carpaccio non contaminé.

Les échantillons artificiellement contaminés ont ensuite été dilués au 1/10 avec de l'eau peptonnée tamponnée (BPW Dilubag 5L réf AEB910305/2, bioMérieux) dans un sac d'enrichissement. Une homogénéisation a été réalisée au malaxeur à pâles, puis les sacs ont été placés à l'étuve à 41,5°C pour 8-24h.

### - Préparation des milieux de culture gélosés :

### Préparation des nanoparticules d'or :

Les nanoparticules de 40 nm sont fabriquées par la réduction du chlorure d'or par du citrate de sodium (méthode décrite par Turkevich and Frens en 1951). Ainsi, les nanoparticules d'or de 20 nm sont fabriquées à l'aide d'une solution de chlorure d'or diluée dans de l'eau distillée, additionnée de citrate trisodique puis portée à ébullition. La présence d'un pic d'absorbance à 517-519 nm permet de s'assurer de la bonne taille des particules. De ces particules de 20 nm sont synthétisées les particules de 40 nm. Pour cela les particules de 20 nm sont diluées en eau distillée puis portées à ébullition avec ajout de citrate trisodique et de chlorure d'or. Le pic d'absorbance est alors observé à 524-526 nm à froid, témoin de l'augmentation de la taille des particules.

### Préparation du conjugué :

Les anticorps utilisés sont l'anticorps 13C4 (ref hybridome ATCC CRL-1794) dirigé contre la toxine STX1 et l'anticorps 9E4H11 dirigé contre la toxine STX2 .

Ce mélange est adsorbé sur les nanoparticules à un pH de 8

### Préparation du milieu de culture:

Le conjugué est ajouté dans la gélose (bioMerieux TBX réf AEB522819) en surfusion à 50°C contenant l'inducteur de toxine à savoir la mitomycine C (Sigma M4287) à 250 ng/mL, afin d'obtenir une concentration en nanoparticules d'une DO 3. Le milieu est ensuite coulé dans les boites puis séché.

### Exemple 2- Détection et confirmation de la présence d'une E. coli O80 productrice de shigatoxines au sein d'une matrice comprenant des entérobactéries selon l'invention

Un prélèvement de l'échantillon « viande hachée » contaminée a été réalisé dans chacun des sacs d'enrichissement et 20µL ont été transférés dans un tube du GENE-UP lysis kit (Ref 414057, bioMérieux) et mis à agiter 5 minutes à l'aide du Vortex Talboys Troemner (161111001, Talboys). Une PCR a été réalisée en transférant 10µL de ce lysat dans un tube PCR GENE-UP STEC stx & eae 2 (Réf 423109, bioMerieux). En cas de résultat positif une seconde PCR a été réalisée en transférant 10µL de lysat dans un tube contenant un mélange réactionnel PCR contenant des amorces et sondes spécifiques du sérogroupe O80 sous forme lyophilisée.

Résultats de la PCR:

| **échantillon** | **contamination** | | | **stx1/stx2** | **eae** | **O80** |
|---|---|---|---|---|---|---|
| A1 | - | - | - | Negative | Negative | Negative |
| A4 | **O80** | 1,5cfu | eae stx2 | Positive | Positive | Positive |
| A5 | | 3 cfu | eae stx2 | Positive | Positive | Positive |

Les PCR réalisées sur l'échantillon « viande hachée » non contaminé artificiellement est négative. La PCR réalisée sur les 2 enrichissements contaminés artificiellement permet de détecter les gènes de virulence stx1 et/ou stx2 et eae, ainsi que le marqueur du serogroupe O80.

Etant donné que le résultat est positif, l'échantillon est ensemencé sur un milieu tel que décrit à l'exemple 1.

Un isolement est réalisé sur le milieu.

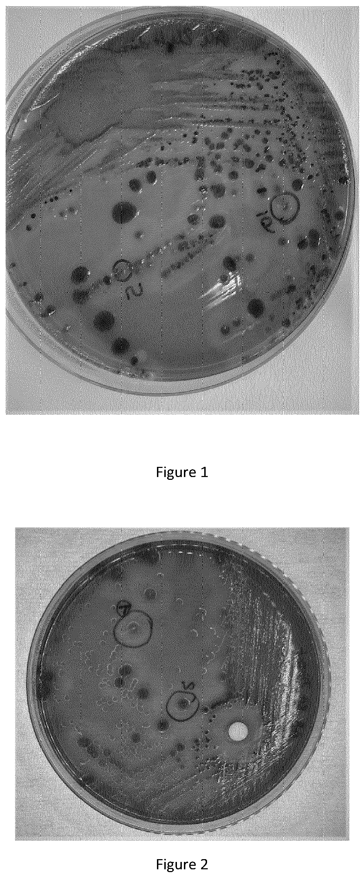

Après incubation des boîtes à 37°C pendant 24h, on observe un halo gris autour de certaines colonies (figure 1). La présence d'un halo permet de confirmer qu'il s'agit d'une STEC.

Une PCR est réalisée sur cette colonie à l'aide d'un mélange réactionnel PCR contenant des amorces et sondes spécifiques des gènes ou portion de gènes eae, stxl, stx2. La PCR donne un résultat positif pour eae et stx2. Une seconde PCR à l'aide d'un mélange réactionnel PCR contenant des amorces et sondes spécifiques du sérogroupe O80 est également réalisée.

La PCR réalisée avec des amorces et sondes spécifiques du sérogroupe O80 réalisée sur la colonie présentant un halo donne bien un résultat positif pour O80. La méthode globale présentée a donc bien permis la détection et la confirmation de la souche O80 (REF H15-66,3 ; CH Robert Debré Paris ) avec laquelle l'échantillon avait été artificiellement contaminé.

### Exemple 3- Détection et confirmation de la présence d'une E. coli O45 productrice de shigatoxines au sein d'une matrice comprenant des entérobactéries selon l'invention

Un prélèvement de l'échantillon de « carpaccio contaminé » a ensuite été réalisé dans chacun des sacs d'enrichissement. Une lyse a été réalisée à l'aide du kit VIDAS ESPT, programme ESP1 (Ref 8300900,bioMérieux). Une PCR a été réalisée en transférant 10µL de ce lysat dans un tube PCR GENE-UP STEC stx 1 & eae (Réf 423109, bioMerieux).

| **échantillon** | **contamination** | | | **stx1/stx2** | **eae** | **O45** |
|---|---|---|---|---|---|---|
| A7 | - | - | - | Negative | Negative | Negative |
| A8 | **O45** | 1.1 cfu | eae stx1 | Positive | Positive | Positive |

En cas de résultat positif une seconde PCR a été réalisée en transférant 10µL de lysat dans un tube contenant un mélange réactionnel PCR contenant des amorces et sondes spécifiques du sérogroupe O45 sous forme lyophilisée.

Les PCR réalisées sur l'échantillon de Carpaccio marinade au basilic non contaminé artificiellement sont négatives. La PCR réalisée sur l'enrichissement contaminé artificiellement permet de détecter les gènes de virulence stx1 et/ou 2 et eae, ainsi que le marqueur du sérogroupe O45.

Etant donné que le résultat est positif, L'échantillon subit une immunosélection avec des anticorps anti-O45. L'échantillon est ensemencé sur un milieu tel que décrit à l'exemple 1.

L'incubation de ces boîtes à 37°C pendant 24h permet la croissance de colonies. On observe un halo gris autour de certaines colonies (figure 2). Une PCR est réalisée sur une colonie présentant un halo à l'aide d'un mélange réactionnel PCR contenant des amorces et sondes spécifiques des gènes ou portion de gènes eae, stxl, stx2. Une seconde PCR à l'aide d'un mélange réactionnel PCR contenant des amorces et sondes spécifiques du sérogroupe O45 est également réalisée.

| **échantillon** | **contamination** | | | **stx1/stx2** | **eae** | **O45** |
|---|---|---|---|---|---|---|
| A8 | **O45** | | eae stx1 | Positive | Positive | Positive |

La PCR réalisée permet bien de confirmer la présence du gène stx, et du gène eae, sur la colonie présentant un halo gris.

Une seconde PCR permettant la détection du sérogroupe O45 est réalisée.

| **échantillon** | **contamination** | | | **O45** |
|---|---|---|---|---|
| A8 | **O45** | | eae stx1 | Positive |

La PCR réalisée avec des amorces et sondes spécifiques du sérogroupe O45 réalisée sur la colonie présentant un halo donne bien un résultat positif pour O45.

La méthode présentée a donc bien permis la détection et la confirmation de la souche O45 ( ATCC BAA-2198) avec laquelle l'échantillon avait été artificiellement contaminé.

## Revendications

1. Méthode de détection et de confirmation d'au moins une *Escherichia Coli* productrice de shigatoxine (STEC) susceptible d'être présente dans un échantillon comprenant des entérobactéries, comprenant les étapes suivantes :
- Effectuer une lyse de l'échantillon permettant la lyse des STEC afin d'obtenir une solution comprenant leurs acides nucléiques
- Mettre en contact la solution d'acides nucléiques avec des amorces permettant d'amplifier au moins le gène ou le fragment de gène stx1 et/ou stx2
- Si au moins un des gènes ou fragments des gènes stx1 et/ou stx2 est amplifié, une partie de l'échantillon est déposé sur un milieu réactionnel gélosé comprenant
▪ au moins un inducteur de toxine,
▪ au moins un conjugué agglutinant formé par au moins un partenaire de liaison spécifique de la protéine STX1 et/ou au moins un partenaire de liaison spécifique de la protéine STX2, couplé(s) à une nanoparticule
- Détecter et confirmer la présence d'au moins une STEC par l'apparition d'un halo sur la gélose autour de ladite STEC.

2. Méthode de détection et de confirmation d'au moins une *Escherichia Coli* productrice de shigatoxine (STEC) selon la revendication 1 dans laquelle,
- si au moins un des gènes ou fragments des gènes stx1 et/ou stx2 est amplifié, la solution d'acides nucléiques est mise en contact avec des amorces permettant d'amplifier au moins le gène ou le fragment de gène des sérogroupes choisis parmi O26, O45, O80, O91, O103, O104, O111, O113, O121, O128ab, O145, O146, O157, O174
- si au moins un des gènes ou fragments des gènes stx1 et/ou stx2 et au moins un sérogroupe choisi parmi O26, O45, O80, O91, O103, O104, O111, O113, O121, O128ab, O145, O146, O157, O174 sont amplifiés, une partie de l'échantillon est déposé sur un milieu réactionnel comprenant
∘ au moins un inducteur de toxine et
∘ au moins un conjugué agglutinant formé par au moins un partenaire de liaison spécifique de la protéine STX1 et/ou au moins un partenaire de liaison spécifique de la protéine STX2, couplé(s) à une nanoparticule.
∘ détecter et confirmer la présence d'au moins une STEC par l'apparition d'un halo sur la gélose autour de ladite STEC.

3. Méthode de détection et de confirmation d'au moins une *Escherichia Coli* productrice de shigatoxine (STEC) selon la revendication 2 dans laquelle
- si au moins un des gènes ou fragments des gènes stx1 et/ou stx2 et au moins un gène ou fragment de gène d'au moins un sérogroupe choisi parmi O26, O45, O80, O91, O103, O104, O111, O113, O121, O128ab, O145, O146, O157, O174. sont amplifiés, une partie de l'échantillon est déposé sur un milieu réactionnel comprenant
∘ au moins un inducteur de toxine et
∘ au moins un conjugué agglutinant formé par au moins un partenaire de liaison spécifique de STX1 couplé à une nanoparticule d'une première nature produisant un halo d'une première couleur lorsqu'il est agglutiné
∘ et/ou au moins un conjugué agglutinant formé par au moins un partenaire de liaison spécifique de STX2 couplé à une nanoparticule d'une première nature produisant un halo d'une première couleur lorsqu'il est agglutiné
∘ et au moins un conjugué agglutinant formé par au moins un partenaire de liaison spécifique dudit sérogroupe identifié par amplification couplé à une nanoparticule d'une deuxième nature produisant un halo d'une deuxième couleur lorsqu'il est agglutiné
- confirmer la présence d'au moins une STEC d'un sérogroupe identifié par l'apparition d'au moins un halo d'une première couleur et d'au moins un halo d'une deuxième couleur autour de la même colonie ou d'au moins un halo résultant du mélange d'une première et d'une deuxième couleur autour de ladite colonie.

4. Méthode de détection et de confirmation d'au moins une *Escherichia Coli entérohémorragique (EHEC)* susceptible d'être présente dans un échantillon comprenant des entérobactéries, comprenant les étapes suivantes :
- effectuer une lyse de l'échantillon permettant la lyse des EHEC afin d'obtenir une solution comprenant leur acides nucléiques
- mettre en contact la solution d'acides nucléiques avec des amorces permettant d'amplifier au moins le gène ou le fragment de gène stx1 et/ou stx2 et eae,
- si au moins un des gènes ou fragments des gènes stx1 et/ou stx2 et au moins un gène ou fragment de gène eae sont amplifiés, une partie de l'échantillon est déposé sur un milieu gélosé comprenant :
∘ au moins un inducteur de toxine
∘ au moins un conjugué agglutinant formé par au moins un partenaire de liaison spécifique de STX1 couplé à une nanoparticule d'une première nature produisant un halo d'une première couleur lorsqu'il est agglutiné
∘ et/ou au moins un conjugué agglutinant formé par au moins un partenaire de liaison spécifique de STX2 couplé à une nanoparticule d'une première nature produisant un halo d'une première couleur lorsqu'il est agglutiné
∘ et au moins un conjugué agglutinant formé par au moins un partenaire de liaison spécifique de EAE couplé à une nanoparticule d'une deuxième nature produisant un halo d'une deuxième couleur lorsqu'il est agglutiné
- confirmer la présence d'au moins une EHEC par l'apparition sur le milieu gélosé :
- d'au moins un halo d'une première couleur
- et d'au moins un halo d'une deuxième couleur
- ou d'au moins un halo résultant du mélange d'une première et d'une troisième couleur autour de ladite souche EHEC.

5. Méthode de détection de confirmation d'au moins une EHEC selon la revendication 4 comprenant la mise en contact de la solution d'acides nucléiques avec des amorces permettant d'amplifier au moins un gène ou fragment de gène d'au moins un sérogroupe choisi parmi O26, O45, O80, O91, O103, O104, O111, O113, O121, O128ab, O145, O146, O157, O174.

6. Méthode de détection et de confirmation selon les revendications précédentes **caractérisée en ce que** la solution d'acides nucléiques est mise en contact avec des amorces permettant d'amplifier le gène ou le fragment de gène aggR.

7. Méthode selon l'une quelconque des revendications précédentes **caractérisée en ce qu'**une étape d'enrichissement de l'échantillon est effectuée préalablement à sa lyse.

8. Méthode de détection et de confirmation selon l'une quelconque des revendications précédentes **caractérisée en ce qu'**une étape d'immuno-sélection d' une *E*. *coli* d'au moins un sérogroupe choisi parmi O26, O45, O80, O91, O103, O104, O111, O113, O121, O128ab, O145, O146, O157, O174 est réalisée avant l'étape de lyse.

9. Méthode de détection et de confirmation selon l'une quelconque des revendications précédentes **caractérisée en ce qu'**une étape d' immuno-sélection d'une E. coli d'au moins un sérogroupe choisi parmi O26, O80, O45, O91, O103, O104, O111, O113, O121, O128ab, O145, O146, O157, O174 est réalisée avant le dépôt sur le milieu réactionnel gélosé.

10. Méthode de détection et de confirmation selon l'une quelconque des revendications précédentes **caractérisée en ce que** la nanoparticule couplée au partenaire de liaison est une nanoparticule colloidale ayant des propriétés optiques.

11. Méthode de détection et de confirmation selon l'une quelconque des revendications précédentes **caractérisée en ce que** la nanoparticule est choisie parmi l'or, l'argent, le cuivre.

12. Méthode de détection et de confirmation selon l'une quelconque des revendications précédentes **caractérisée en ce que** les nanoparticules ont une taille comprise entre 10 et 200 nm, préférentiellement entre 20 et 90 nm.

13. Méthode de détection et de confirmation selon l'une quelconque des revendications précédentes **caractérisée en ce que** l'inducteur de toxine est un antibiotique.

14. Méthode de détection et de confirmation selon l'une quelconque des revendications précédentes **caractérisée en ce que** l'inducteur de toxine est la ciprofloxacine à une concentration comprise entre 0,005 et 0,030 mg/l.

15. Méthode de détection et de confirmation selon l'une quelconque des revendications précédentes **caractérisée en ce que** l'inducteur de toxine est la mitomycine C à une concentration comprise entre 0,10 mg/l à 0 ,50 mg/l.
